# FASCICULE DE BREVET EUROPEEN

(11) **EP 2 628 524 B1**
(45) Date de publication et mention de la délivrance du brevet: **29.05.2019**
(21) Numéro de dépôt: 12155273.1
(22) Date de dépôt: 14.02.2012
(51) Int. Cl.: B01D 53/053, A61M 16/10, F24F 3/12

(54) **Installation de production sur site de gaz médical**
Vor Ort Produktionsanlage von medizinischem Gas
Facility for on-site production of medical gas

(43) Date de publication de la demande: 21.08.2013
(73) Titulaire: Air Liquide Medical G.m.b.H., 40235 Düsseldorf (DE)
(72) Inventeur: Maamar, Kais, 45128 Essen (DE); Bongers, Karsten, 47800 Krefeld (DE); Franken, Hartmut, 47829 Krefeld (DE); Neu, Peter, 47249 Duisburg (DE); Sommier, Vincent, 75014 Paris (FR)
(74) Mandataire: Pittis, Olivier

(56) Documents cités:
- EP-A1- 0 537 612
- EP-A1- 0 864 818
- EP-A2- 1 004 341
- EP-A2- 1 393 794
- FR-A1- 2 658 155
- US-A- 5 809 999
- US-A- 5 915 834
- US-A1- 2004 079 359
- US-A1- 2004 103 895
- US-A1- 2007 214 960

## Description

L'invention concerne une installation de production sur site d'air médical, c'est-à-dire au sein d'un bâtiment hospitalier ou analogue.

L'air médical utilisé dans les hôpitaux, les cliniques, les centres de soins, les unités d'urgence ou de secours, ou analogues pour la respiration des patients est un médicament dont la composition est donnée par la pharmacopée européenne.

Plus précisément, l'air médical est de l'air ambiant comprimé à une pression supérieure à la pression atmosphérique, typiquement à plusieurs bar, voire à plusieurs dizaines ou même centaines de bar, et contenant (en volume) de 20,4% à 21,4% d'oxygène, au maximum 500 ppm de CO₂, au maximum 5 ppm de CO, au maximum 1 ppm de SO₂, au maximum 2 ppm de NO et de NO₂, au maximum 67 ppm d'eau et au maximum 0,1 mg/m³ d'huile ; les vapeurs d'huile éventuellement présentes provenant essentiellement de la compression de l'air.

A noter que les composés susmentionnés autres que l'oxygène (COx, NOx, eau, huile...), sont en fait des impuretés dont la présence est tolérée dans les limites de la pharmacopée mais qui idéalement n'y sont pas présentes.

L'air médical contient en outre de l'azote et peut aussi contenir d'autres composés, tel l'argon.

Actuellement, l'air médical est fourni aux hôpitaux ou analogues sous trois formes, à savoir selon le cas :
- une livraison directe sous forme d'air comprimé, par exemple à une pression absolue de 200 à 300 bar, dans des cylindres, c'est-à-dire des bouteilles ou bonbonnes de gaz, ou des cadres regroupant plusieurs bouteilles ;
- une production sur site par mélange d'oxygène et d'azote de manière à créer des mélanges azote/oxygène, et
- une production directement sur site à partir d'air ambiant traité notamment par compresseurs et chaines de filtration/purification.

Parmi celles-ci, la production d'air directement sur site, par compresseurs et chaines de filtration est la solution la plus répandue. Un tel procédé est par exemple décrit dans le document EP-A-864818.

L'air ambiant est aspiré, comprimé par des compresseurs à une plage de pression allant de 1 bar à 80 bar relatifs. Cet air comprimé est ensuite filtré, c'est-à-dire purifié, via une ou plusieurs étapes de traitement, par exemple par un jeu de filtres et/ou par mise en oeuvre d'un procédé à pression modulée ou PSA (pour *Pressure Swing Adsorption*).

L'air médical ainsi produit peut être stocké dans une ou plusieurs capacités-tampons intermédiaires, puis envoyé dans le réseau de canalisations qui parcourt le bâtiment hospitalier pour approvisionner les salles de soins, les chambres ou autres en air médical. Il est bien évidemment possible, voire indispensable dans certains cas, de procéder à une détende intermédiaire du gaz, par exemple pour passer d'une pression de 10 bar environ dans la capacité de stockage à une pression de 5 ou 8 bars au sein du réseau.

On palie en général à toute rupture d'approvisionnement en air médical, grâce à de l'air médical issu d'une source de réserve ou de secours ou l'air est conservé sous forme gazeuse.

Les autres gaz médicaux utilisés dans les hôpitaux ou les centres de soins, tel l'oxygène, sont aussi fournis de manière analogue à l'air. Les compositions de ces autres gaz sont également données par la pharmacopée européenne.

Ainsi, l'oxygène peut être également produit sur site par procédé PSA en utilisant des adsorbants spécifiques, telles des zéolites X échangées au lithium, permettant de retenir l'azote contenu dans l'air et de produire ainsi de l'oxygène gazeux ayant une pureté typiquement supérieure à 90%, voire à 93% en volume, comme connu du document EP-A-297542.

Toutefois, les procédés de production d'air médical ou d'autres gaz médicaux utilisés sur site (encore appelés procédés *on site*) présentent certains inconvénients.

Tout d'abord, ces procédés ne permettent pas un contrôle aisé de la fiabilité du processus de fabrication.

Ainsi, lorsqu'une unité de production sur site d'air médical fonctionne de façon autonome, le processus de fabrication n'est pas suivi en continu et les interventions sur l'installation se font sur une base d'une planification, c'est-à-dire une maintenance préventive, ou lorsqu'une erreur ou un problème survient sur l'installation, c'est-à-dire une maintenance curative.

Ces interventions sont donc réalisées indépendamment de l'état de l'installation et de sa fiabilité, ce qui n'est pas optimal car elles sont réalisées ou trop tôt, donc sans nécessité réelle, ou trop tard, donc avec une incidence sur le processus de production et éventuellement sur le produit final.

Ensuite, on assiste à des blocages de polluants dans la canalisation principale lorsque le gaz produit n'est pas conforme. En fait, dans les installations existantes, l'électrovanne de contrôle est une électrovanne dite « à 2 voies » qui est agencée sur la ligne principale.

Si, elle permet d'arrêter une éventuelle pollution en amont de la vanne, cette pollution reste cependant bloquée dans la ligne principale et nécessite une purge totale du système en amont de la vanne. Ceci n'est pas idéal car il nécessite un arrêt de la production de gaz et une intervention manuelle.

Par ailleurs, en cas de courtes ruptures de fourniture d'air dues par exemple à une brève contamination à l'entrée, la source de secours est directement sollicitée. Or, cela pose problème car le volume de secours est limité et donc si la fréquence des ruptures de fourniture est importante, alors on risque d'assécher la source de secours. En d'autres termes, il serait très bénéfique de pouvoir éviter cet inconvénient en réduisant les sollicitations de la source de secours de manière à augmenter son autonomie au fil du temps.

Enfin, l'air produit par les procédés et installations actuels, n'est en général ni analysé, ni validé du point de vue pharmaceutique, ce qui peut poser d'évidents problèmes de conformité et de qualité. D'ailleurs, lorsqu'il est analysé, en cas de « non-conformité », on assiste habituellement soit à une interruption immédiate de la production et passage sur une source d'air de secours, ce qui peut avoir pour conséquence une utilisation abusive de l'air de secours susceptible de conduire à une éventuelle rupture totale de la fourniture en air ; soit à une continuité de fourniture d'un air non conforme et déclenchement en parallèle d'une alerte pour avertir l'utilisateur qui doit alors intervenir de façon manuelle. On comprend que ces solutions ne sont pas non plus idéales.

Le document EP-1393794 enseigne un système concentrateur d'oxygène comprenant une unité de purification de gaz de type PSA permettant de produire soit de l'oxygène, soit de l'air médical, à partir d'air ambiant. L'oxygène produit est stocké dans une capacité agencée en aval de l'unité PSA sur une ligne principale reliant l'unité PSA à une sortie d'oxygène. Un dispositif de pilotage contrôle le système et, en particulier, l'unité PSA et reçoit des informations d'un capteur de pression agencé sur la ligne principale en aval de la capacité. Le dispositif de pilotage est relié par ailleurs à des capteurs de CO, d'humidité et d'oxygène (140) agencé sur la ligne d'air médical, en aval du PSA produisant l'air médical.

Par ailleurs, le document US-A-5,809,999 enseigne un système de fourniture d'oxygène aux pilotes et passagers d'un avion qui comprend un dispositif de production d'oxygène à partir d'air ambiant. L'oxygène produit est stocké dans deux capacités-tampons agencées en série. L'entrée de gaz dans ces capacités est contrôlée en fonction de la teneur en oxygène en sortie de production par une unité centrale qui commande deux vannes prévues en amont des capacités de stockage.

En outre, le document US-A-2004/0103895 porte, quant à lui, sur un dispositif de production d'oxygène, dans lequel on procède aussi à une vérification de la teneur en oxygène entre deux capacités de stockage agencées en série.

Enfin, le document FR-A-propose aussi un système de fourniture d'oxygène aux pilotes et passagers d'un avion où l'oxygène produit est envoyé vers des bouteilles de stockage agencées en parallèle les unes des autres. Une unité centrale de pilotage est prévue pour piloter la production du gaz. Des dispositifs de type régulateur à la demande ou manodétendeur, sont agencés en aval des bouteilles de gaz.

En définitive, il n'existe actuellement aucune méthode de validation de l'air produit sur site permettant de garantir que l'air produit est bien conforme aux spécifications requises et qui permette d'assurer une fourniture en air médicale efficace et fiable.

En d'autres termes, le problème qui se pose est de proposer une installation de production sur site et en continu d'un gaz médicament, en particulier d'air médical, conformément aux bonnes pratiques de fabrication ou GMP (pour *Good Manufacturing Practice*) qui permet notamment :
- une supervision de la fiabilité du processus de fabrication avec détection rapide de toute anomalie,
- un contrôle des différentes étapes de production et en particulier de la production finale, avec pour chaque étape, une possibilité de purge permettant ainsi d'arrêter toute contamination ou non-conformité du gaz produit, en particulier d'air médical, et/ou
- de réduire l'utilisation des sources de secours à un niveau minimal.

La solution de l'invention est une installation de production sur site de gaz médical, en particulier d'air médical, comprenant :
- une unité de purification de gaz apte à produire un gaz purifié à partir d'un gaz d'alimentation, tel de l'air ambiant,
- une ligne principale de gaz alimentée en gaz par l'unité de purification de gaz et comprenant, en série, une première capacité de stockage de gaz et une deuxième capacité de stockage de gaz purifié, et
- une ligne secondaire connectée fluidiquement à la ligne principale, en aval de première capacité de stockage de gaz, et comprenant une troisième capacité de stockage de gaz,
- au moins un dispositif d'analyse de gaz agencé de manière à déterminer la teneur du gaz en au moins une impureté donnée, en aval de la deuxième et de la troisième capacité,
- une première électrovanne agencée sur la ligne principale de gaz entre la première capacité de stockage de gaz et la deuxième capacité de stockage de gaz,
- une deuxième électrovanne agencée sur la ligne secondaire de gaz entre la première capacité et la troisième capacité de stockage de gaz,
- une troisième électrovanne agencée sur la ligne principale de gaz en aval de la deuxième capacité de stockage de gaz,
- une quatrième électrovanne agencée sur la ligne secondaire de gaz en aval de la troisième capacité de stockage de gaz, et
- un dispositif de pilotage commandant l'ouverture et/ou la fermeture desdites électrovannes en réponse à au moins un signal de mesure de teneur en impureté reçu dudit au moins un dispositif d'analyse de gaz.

Selon le cas, l'installation de l'invention peut comprendre l'une ou plusieurs des caractéristiques techniques suivantes :
- une électrovanne à trois voies est agencée sur la ligne principale de gaz entre l'unité de purification de gaz et la première capacité de stockage.
- l'électrovanne à trois voies est connectée à l'atmosphère via une ligne d'échappement, et est commandée par le dispositif de pilotage. De préférence, on dévie le gaz présent dans la canalisation principale, en amont de l'électrovanne à trois voies, vers la ligne d'échappement et on stoppe simultanément l'envoi de gaz vers la première capacité de stockage, lorsque la teneur en impuretés mesurée dans la canalisation principale est supérieure ou égale à une teneur-seuil préfixée. On opère ensuite un balayage gazeux de la partie de canalisation contaminée par des impuretés avec du gaz purifié et on évacue à l'atmosphère le flux gazeux ainsi généré, via la ligne d'échappement.
- la ligne principale et la ligne secondaire alimentent un ou plusieurs sites utilisateurs de gaz, de préférence un réseau de canalisations.
- elle comporte un premier dispositif d'analyse de gaz ayant une première ligne de mesure raccordée fluidiquement à la ligne principale, en amont de la première capacité de stockage.
- le dispositif de pilotage commande l'électrovanne à trois voies en réponse aux mesures faites par le premier dispositif d'analyse de gaz.
- elle comporte un deuxième dispositif d'analyse de gaz ayant au moins une deuxième ligne de mesure raccordée fluidiquement, directement ou indirectement, à la ligne principale et à la ligne secondaire, en aval de la deuxième capacité de stockage et de la troisième capacité de stockage, respectivement.
- elle comporte une première ligne de purge raccordée fluidiquement à la ligne principale en aval de la deuxième capacité.
- elle comporte une deuxième ligne de purge raccordée fluidiquement à la ligne secondaire en aval de la troisième capacité.
- le deuxième dispositif d'analyse de gaz est raccordé fluidiquement à la ligne principale et à la ligne secondaire, de préférence en aval des deuxième et troisième capacités.
- la première ligne de purge et/ou la deuxième ligne de purge sont raccordées fluidiquement à la ligne d'échappement.
- la première ligne de purge comprend une cinquième électrovanne et/ou la deuxième ligne de purge comprend une sixième électrovanne.
- la deuxième ligne de mesure comprend une septième et/ou une huitième électrovanne.
- lesdites cinquième, sixième, septième et/ou huitième électrovannes sont commandées par le dispositif de pilotage.
- une unité de compression de gaz alimente l'unité de purification de gaz avec un gaz à purifier, tel de l'air, comprimé à une pression supérieure à 1 bar absolu.
- l'unité de compression de gaz comprend au moins un compresseur à vis, à pistons, à spirales ou à membranes.
- l'unité de purification de gaz comprend au moins un adsorbeur contenant au moins un lit d'au moins un matériau adsorbant.
- l'unité de purification de gaz comprend au moins deux adsorbeurs fonctionnant de manière alternée, de préférence selon des cycles PSA.
- le dispositif de pilotage commande en outre l'unité de purification de gaz et/ou l'unité de compression de gaz.
- le dispositif de pilotage coopère avec le premier et/ou le deuxième dispositif d'analyse de gaz.
- le gaz à purifier est de l'air ambiant.
- le gaz purifié est de l'air médical ou de l'oxygène médical, c'est-à-dire un gaz conformes aux spécifications de la pharmacopée européenne.

On décrit aussi un procédé de pilotage d'une installation de production sur site de gaz médical, en particulier d'air médical, comprenant une ligne principale de gaz comprenant, en série, une première capacité de stockage de gaz et une deuxième capacité de stockage de gaz purifié, et une ligne secondaire connectée fluidiquement à la ligne principale, en aval de première capacité de stockage de gaz, et comprenant une troisième capacité de stockage de gaz, la ligne principale de gaz et la ligne secondaire alimentant chacun au moins un site utilisateur de gaz, en particulier une installation selon l'invention telle que décrite ci-avant, comprenant les étapes de :
a) alimenter ledit au moins un site utilisateur avec du gaz purifié ayant une composition souhaitée provenant de la troisième capacité,
b) alimenter la deuxième capacité avec du gaz provenant de la première capacité de stockage de gaz,
c) stopper l'alimentation de la deuxième capacité avec le gaz provenant de la première capacité de stockage de gaz lorsque la deuxième capacité est remplie de gaz,
d) analyser la composition du gaz stocké dans la deuxième capacité pour déterminer si la composition du gaz stocké dans la deuxième capacité est conforme à la composition souhaitée,
e) en fonction du résultat de l'étape d), envoyer le gaz stocké dans la deuxième capacité:
   i) soit vers l'atmosphère lorsque la composition analysée n'est pas conforme à la composition souhaitée,
   ii) soit vers ledit au moins un site utilisateur lorsque la composition analysée est conforme à la composition souhaitée.

Selon le cas, le procédé peut comprendre l'une ou plusieurs des caractéristiques techniques suivantes :
- à l'étape e) ii), on commence à alimenter ledit au moins un site utilisateur avec du gaz stocké dans la deuxième capacité, après un arrêt de l'alimentation dudit site utilisateur avec du gaz issu de la troisième capacité.
- à les étapes a) et b) sont au moins en partie simultanées.
- à l'étape e) i), on vide totalement ou quasi-totalement la deuxième capacité et on répète les étapes b) à e).
- les deuxième et troisième capacités fonctionnent de manière à subir alternativement les étapes a) à e).
- le gaz alimentant les deuxième et troisième capacités est produit par une unité de purification de gaz comprenant au moins un adsorbeur contenant au moins un lit d'au moins un matériau adsorbant, de préférence au moins deux adsorbeurs fonctionnant de manière alternée, ledit au moins un adsorbeur étant alimenté en gaz à purifier par une unité de compression de gaz.
- à l'étape d), on analyse la composition du gaz au moyen d'au moins un dispositif d'analyse de gaz agencé de manière à déterminer la teneur du gaz en au moins une impureté donnée, en aval de la deuxième et de la troisième capacité, de préférence la ou les impuretés sont choisies parmi les NOx, les SOx, les COx, la vapeur d'eau et les vapeurs d'hydrocarbures, notamment les vapeurs d'huile.
- les étapes a) à e) sont commandées par un dispositif de pilotage agissant sur des vannes agencées sur la ligne principale de gaz, la ligne secondaire, sur la première ligne de purge raccordée fluidiquement à la ligne principale en aval de la deuxième capacité, et/ou la deuxième ligne de purge raccordée fluidiquement à la ligne secondaire en aval de la troisième capacité, ainsi que sur la/les lignes d'analyse.
- le gaz produit est de l'air médical ou de l'oxygène médical, de préférence de l'air médical.
- l'air médical produit contient (en volume) de 20,4% à 21,4% d'oxygène, au maximum 500 ppm de CO₂, au maximum 5 ppm de CO, au maximum 1 ppm de SO₂, au maximum 2 ppm de NO et de NO₂, au maximum 67 ppm d'eau, au maximum 0,1 mg/m³ d'huile, et de l'azote.

La présente invention va maintenant être décrite plus en détail en références aux Figures annexées parmi lesquelles :
- la Figure 1 représente un schéma de principe d'une installation 100 de production sur site de gaz médicaux selon l'invention,
- la Figure 2 illustre les différentes étapes du procédé de pilotage de l'installation de la Figure 1.

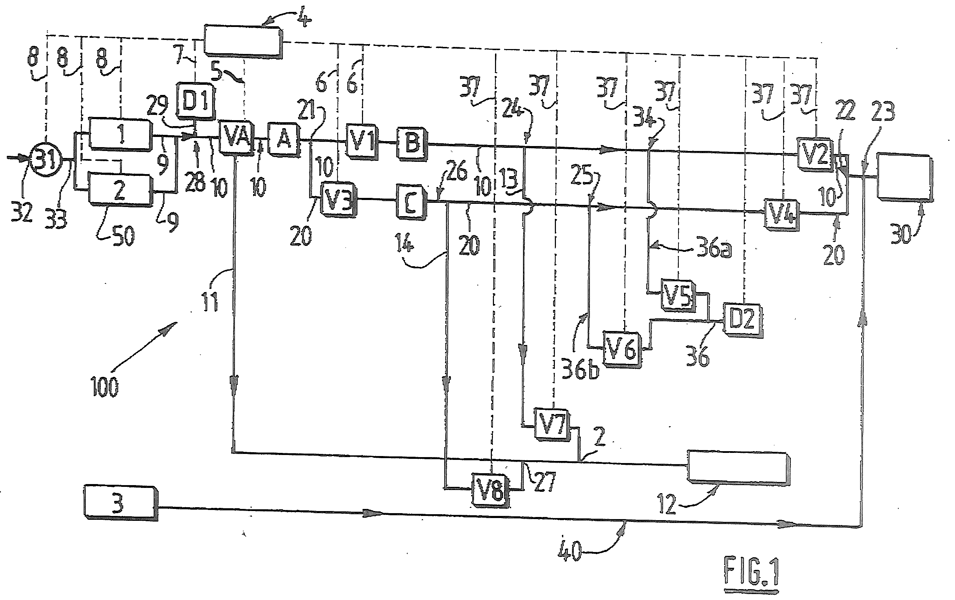

La Figure 1 est un schéma de principe d'un mode de réalisation d'une installation 100 de production sur site de gaz médicaux commandée par un procédé de pilotage, laquelle est raccordée au réseau de canalisations 30 d'un bâtiment hospitalier ou analogue.

Le gaz produit ici est de l'air médical, c'est-à-dire de l'air purifié répondant aux spécifications de la pharmacopée européenne susmentionnée. Toutefois, une telle installation 100 peut être utilisée pour fabriquer d'autres gaz médicaux, par exemple de l'oxygène médical à partir d'air ambiant.

Plus précisément, selon ce mode de réalisation, l'installation de production 100 sur site d'air médical comprend une unité de purification 50 de gaz alimentée par une unité de compression de gaz 31, c'est-à-dire une ou plusieurs compresseurs d'air aspirant de l'air ambiant (à pression atmosphérique : 1 atm) par leur entrée d'alimentation 32 et délivrant de l'air comprimé à une pression supérieure à la pression atmosphérique, par exemple à une pression comprise entre 1 bar et 80 bar absolus. Ce ou ces compresseurs 31 peuvent être un ou des compresseurs à vis, à pistons, à spirales ou à membranes.

L'air comprimé alimente l'unité de purification de gaz 50 qui comprend ici deux adsorbeurs 1, 2 fonctionnant en parallèle selon des cycles de type PSA (Pressure Swing Adsorption), c'est-à-dire que l'un est en phase de production pendant que l'autre est en phase de régénération, et inversement. Typiquement, la durée d'un cycle de production est comprise entre 1 et 30 minutes environ, de préférence de moins de 10 à 15 minutes.

Ces adsorbeurs 1, 2 contiennent chacun au moins un lit d'au moins un matériau adsorbant, par exemple des matériaux adsorbants de type zéolites, alumines, charbon actif, gel de silice ou tout autre tamis moléculaire apte à stopper des impuretés présentes dans l'air ambiant.

Pour améliorer la purification de l'air et l'élimination des impuretés, on peut également prévoir la présence de filtres, par exemple un filtre à charbon actif et/ou d'autres filtres aptes à stopper tout ou partie des impuretés à éliminer.

L'unité de purification de gaz 50 peut aussi comprendre, selon le mode de réalisation considéré, un adsorbeur unique ou plus de 2 adsorbeurs, 1, 2 par exemple au moins 3 adsorbeurs.

Ce type d'adsorbeurs 1, 2 et de cycle PSA sont bien connus et, à ce titre, on peut d'ailleurs se reporter par exemple aux documents EP-A-716274, EP-A-718024, EP-A-922482, GB-A-1551348, EP-A-930089.

Dans tous les cas, les adsorbeurs 1, 2 permettent d'éliminer tout ou partie des impuretés éventuellement présentes dans l'air ambiant à purifier ou qui y ont été introduites lors de la compression en 31, en particulier la vapeur d'eau, les vapeurs d'huile, les SOx, COx et/ou les NOx, de manière à produire de l'air de qualité médicale ou air médical conforme à la pharmacopée européenne.

Ensuite, l'air purifié (ou tout autre gaz médical) produit par l'unité de purification de gaz 50 est récupéré dans des conduites de sortie 9 qui alimentent une ligne principale 10 d'acheminement de gaz, c'est-à-dire une canalisation ou un tuyau d'amenée de gaz, apte à et conçue pour véhiculer l'air purifié ainsi produit jusqu'à une première capacité A de stockage, c'est-à-dire une capacité-tampon, où l'air médical purifié peut être stocké et homogénéisé, avant d'être envoyé vers un ou plusieurs sites utilisateurs 30, tel un réseau de canalisations de gaz parcourant un bâtiment hospitalier pour amener l'air médical dans les différentes salles où il doit être utilisé, comme les salles de soins, les salles d'urgence, les salles de réveil, les chambres ou tout autre emplacement.

La ligne principale de gaz 10 relie donc fluidiquement la (ou les) sortie 9 de l'unité de purification de gaz 50 à ladite première capacité A de stockage de manière à l'alimenter en air purifié provenant des deux adsorbeurs 1, 2 de l'unité de purification de gaz 50.

Le fonctionnement du ou des compresseurs 31 et les cycles de purification ayant lieu dans l'unité de purification de gaz 50 sont commandés et contrôlés par un dispositif de pilotage 4, par exemple un automate programmable ou analogue, relié à l'unité de purification de gaz 50 par des liaisons électriques 8, tels des câbles électriques.

A noter toutefois que, de façon générale, les communications entre les différents éléments et dispositifs de l'installation, notamment avec l'automate 4, pourraient aussi se faire par liaisons non filaires, par exemple via un ou des dispositifs ou systèmes émetteurs sans fil de type radiofréquence (RF), Bluetooth, Zigbee, wifi, GSM ou GPRS, et une ou des antennes réceptrices permettant d'assurer des transmissions sans fil des données adaptées au type d'émetteur utilisé.

De préférence, l'automate 4 ou similaire est programmé en fonction des besoins du site hospitalier considéré et peut être reprogrammé si les besoins du site change par exemple.

Afin de réguler et contrôler le cheminement du gaz dans la ligne principale de gaz 10, une électrovanne VA est agencée sur ladite ligne principale 10 entre l'unité de purification de gaz 50 et la première capacité A de stockage. L'électrovanne VA est également commandée par le dispositif de pilotage 4 via une liaison électrique 5, tel un câble électrique.

La vanne VA est une électrovanne à trois voies dont l'une des voies est raccordée fluidiquement via une ligne d'échappement 11 à l'atmosphère ambiante (en 12) où se trouve de préférence un dispositif d'échappement à l'atmosphère, tel qu'une soupape d'échappement (non représenté), et dont les deux autres voies sont connectées fluidiquement à la ligne principale 10.

L'air produit par l'unité de purification de gaz 50 traverse donc deux des trois voies de l'électrovanne VA, c'est-à-dire les première et deuxième voie de l'électrovanne VA, lorsqu'elle traverse normalement ladite électrovanne VA, en direction de la capacité tampon A où le gaz purifié peut être stocké.

Par contre, en cas de contamination de la ligne 10 en amont de la vanne VA, cette pollution peut être chassée facilement et efficacement de la portion de conduit contaminée de la ligne principale 10, sans nécessiter une purge totale du système en amont de la vanne VA.

Ceci se fait classiquement en procédant à un balayage de la portion de conduit polluée par des impuretés, avec de l'air pur produit par l'unité de purification de gaz 50. Le flux gazeux entrainant les impuretés est alors évacué via la troisième voie de l'électrovanne VA, vers l'atmosphère par la ligne d'échappement 11 à l'atmosphère ambiante. En d'autres termes, l'air produit par l'unité de purification de gaz 1, 2 va alors entraîner avec lui les polluants et ceux-ci seront rejetés à l'atmosphère (en 12).

L'électrovanne VA à 3 voies permet donc non seulement de bloquer toute pollution éventuelle sur la ligne principale 10 pour la confiner en amont de l'électrovanne VA mais aussi d'évacuer ensuite cette pollution de la ligne principale 10 vers l'extérieur (en 12) et purger ainsi la ligne principale 10 en amont de la vanne VA.

Ceci permet d'éviter d'arrêter entièrement le processus de production et d'avoir recours au secours 3, c'est-à-dire d'une réserve de gaz pur, en cas de pollution temporaire de l'air aspiré ou créée par la ligne de production.

Par ailleurs, la première capacité-tampon A permet de prendre le relai de livraison du gaz purifié, tel l'air médical, lorsque la vanne VA est en position échappement, c'est-à-dire lorsqu'une purge de la ligne 10 est en cours, de manière à, là encore, réduire la fréquence de recours à la source secours 3.

La première capacité A permet également de protéger l'unité de production 50 des pics de consommation, c'est-à-dire les pics de demande de la part des sites utilisateurs 30, et d'homogénéiser l'air produit par l'unité de production 50.

La surveillance de la composition du gaz produit, tel l'air purifié, délivré par l'unité de production 50 se fait au moyen d'un premier dispositif d'analyse de gaz Dl, tel une baie d'analyse ou tout autre analyseur de gaz adapté, dont la ligne de mesure 29 est raccordée fluidiquement (en 28) à la ligne principale 10, en amont de l'électrovanne VA à 3-voies.

Ce dispositif d'analyse de gaz D1 est relié au dispositif de pilotage 4 via une liaison électrique 7, tel un câble électrique ou analogue, de manière à lui transmettre des signaux de mesure et éventuellement autres informations. En fonction des signaux reçus, le dispositif de pilotage 4 peut rétroagir sur l'électrovanne VA à 3-voies et préférentiellement les autres éléments de l'installation, tel que unité de production 50, compresseur 31..., pour déclencher une purge de la ligne 10 lorsqu'une pollution est détectée.

Plus précisément, pour évaluer la fiabilité du procédé et de l'installation de production, la qualité de l'air produit est analysée via la baie d'analyse Dl, notamment les teneurs en H₂O, CO₂ et vapeur d'huile, de manière à s'assurer que l'air produit est conforme à une composition souhaitée, notamment celle de la pharmacopée européenne susmentionnée.

Des variables de suivi complémentaires (par exemple température, pression, vibrations...) sont par ailleurs collectées à partir de l'installation. Les résultats d'analyse ainsi que les variables de suivi sont par la suite traités par le dispositif de pilotage 4, tel un automate, sur une base statistique ou SPC (pour *Statistical Process Control*) pour définir la fiabilité du procédé de production.

Le traitement des données se fait pour chacune des lignes de production, c'est-à-dire pour chaucun des adsorbeurs 1, 2 de l'unité de production 50 ainsi que les compresseurs 31, sur la base d'éléments classiques de contrôle, tels qu'indicateurs d'aptitude du procédé de production, carte de contrôle des variables, moyenne, limites de contrôle, analyse de tendance des variables...

A partir des résultats et des paramètres prédéfinis, on peut alors déterminer si le procédé de fabrication est fiable ou pas.

Si le procédé de fabrication n'est plus fiable, la ligne de production concernée, c'est-à-dire l'adsorbeur 1 ou 2 et le compresseur 31 associé, est arrêtée et la deuxième ligne, c'est-à-dire l'autre adsorbeur 2 ou 1, respectivement, prend le relai pour produire de l'air purifié, alors que l'autre ligne est régénérée, réinitialisée et/ou subit une opération de maintenance.

En cas de non-fiabilité de la seconde ligne, le système bascule alors sur la source de secours 3. En effet, l'installation comprend aussi une ligne de secours 40 reliant fluidiquement une source de secours 3, tel un réservoir de secours contenant de l'air médical, aux sites utilisateur de gaz 30, directement ou indirectement, c'est-à-dire en venant se raccorder en 23 à la ligne principale de gaz 10 ou à une ligne secondaire 20.

La ligne secondaire 20 est en fait une autre ligne de gaz agencée en parallèle de la ligne principale 10 et servant de passage alternatif au gaz venant de la capacité A par exemple et/ou de l'unité de production 50.

En fait, en fonctionnement normal, le gaz envoyé aux sites utilisateurs 30, tel un réseau de conduites de gaz au sein d'un bâtiment hospitalier, est fourni alternativement par les capacités de stockage de gaz B et C selon le procédé cyclique décrit ci-après.

Ces capacités B, C servent en effet à alimenter alternativement le ou les sites utilisateurs 30 en gaz respiratoire, c'est-à-dire à fournir de l'air médical en alternance, c'est-à-dire que pendant qu'une capacité (B par exemple) est en cours de remplissage ou d'analyse de conformité, la deuxième (C respectivement) délivre le gaz au réseau hospitalier 30, ou inversement.

Dit autrement, la ligne principale de gaz 10 se ramifie (en 21) en aval de la première capacité A en la ligne secondaire 20. Celle-ci 20 vient donc se connecter fluidiquement, de son côté amont (en 21) à ladite ligne principale 10 et, par son extrémité aval à au moins un site utilisateur de gaz (30), directement ou indirectement, c'est-à-dire en venant se raccorder (en 22) à la ligne principale de gaz 10.

La ligne 10 comprend une deuxième capacité-tampon B de stockage de gaz purifié située entre la première capacité A et le ou les sites utilisateur de gaz, tel un réseau de canalisations 30 d'un bâtiment hospitalier, et la ligne secondaire 20 comprend, quant à elle, une troisième capacité C de stockage de gaz purifié.

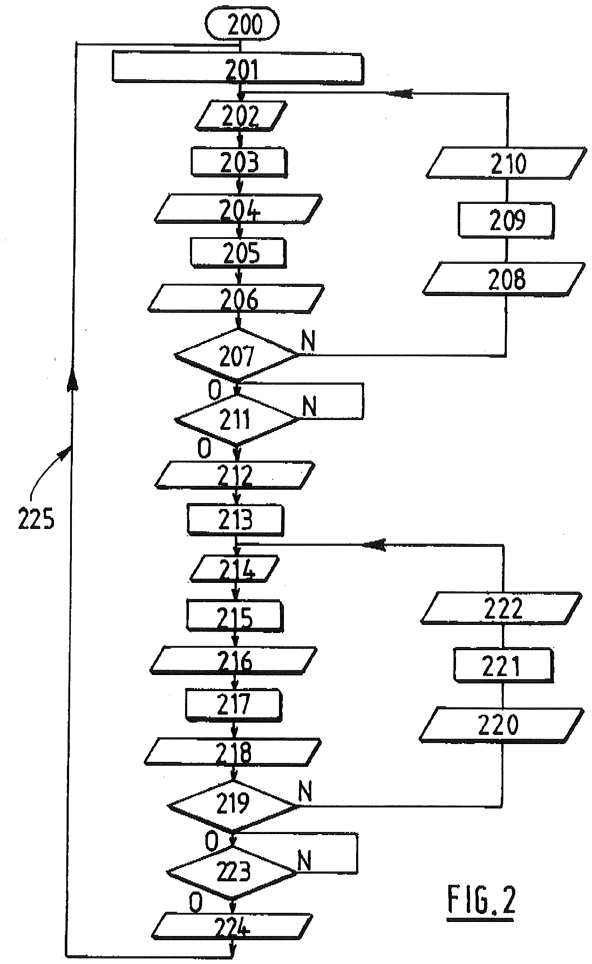

Comme déjà dit, les capacités de stockage B, C servent à alimenter le ou les sites utilisateurs 30 selon une séquence ou cycle de procédé bien déterminé, illustré en Figure 2, en garantissant que l'air envoyé aux sites utilisateurs 30 est bien conforme aux spécifications de la pharmacopée, c'est-à-dire qu'il s'agit bien d'air de qualité médical (air médical), et, dans le cas contraire, de prendre les mesures nécessaires pour évacuer tout air non conforme vers l'atmosphère, comme détaillé ci-après.

D'une façon générale, une maintenance de l'installation 100 est déclenchée par l'automate 4 sur une base prévisionnelle lorsque les paramètres de production d'une ou des deux unités de production 1, 2 atteignent un seuil prédéterminé.

On note par ailleurs que des électrovannes à 2-voies sont agencées sur la ligne principale 10 et la ligne secondaire 20. Plus précisément, une première électrovanne V1 est agencée entre la première capacité A et la deuxième capacité B, et une deuxième électrovanne V3 est agencée entre la première capacité A et la troisième capacité C de stockage de gaz purifié. En outre, une troisième électrovanne V2 agencée en aval de la deuxième capacité B et une quatrième électrovanne V4 est agencée en aval de la troisième capacité C. Ces électrovannes à 2-voies sont commandées par le dispositif de pilotage 4 via des liaisons électriques 6, 37, tels des câbles ou analogues, et permettent de contrôler le passage du gaz dans les lignes 10, 20 et donc à sectionner les canalisations 10, 20 en tronçons bien déterminés, et aussi à gérer et/ou à piloter les entées et/ou sorties de gaz des capacités B et C.

L'installation 100 comprend par ailleurs une première ligne de purge 13 raccordée fluidiquement, par son extrémité amont 24, à la ligne principale 10, de préférence en aval de la deuxième capacité B, et, par son extrémité aval 25, à la ligne d'échappement 11, ainsi qu'une deuxième ligne de purge 14 raccordée fluidiquement, par son extrémité amont 26, à la ligne secondaire 20, de préférence en aval de la troisième capacité C, et, par son extrémité aval 27, à la ligne d'échappement 11.

La première ligne de purge 13 comprend une cinquième électrovanne V7 et la deuxième ligne de purge 14 comprend une sixième électrovanne V8 servant à contrôler le passage du gaz vers la ligne d'échappement 11. Le dispositif de pilotage 4 commande aussi les électrovannes V7, V8 via des liaisons électriques 37.

Ces lignes de purge 13, 14 permettent de purger les portions de lignes 10, 20 ainsi que les capacités B et C, respectivement, situées en amont des électrovannes V2 et V4, respectivement.

Un deuxième dispositif d'analyse de gaz D2, telle une baie d'analyse ou analogue, est prévu et comporte au moins une deuxième ligne de mesure 36, se ramifiant en deux sous-tronçons 36a, 36b, est raccordée fluidiquement (en 34, 35) à la ligne principale 10 et à la ligne secondaire 20, notamment par le biais des sous-tronçons 36a, 36b. De préférence, la deuxième ligne de mesure 36, 36a, 36b comprend une septième V5 et/ou une huitième V6 électrovanne.

Ce deuxième dispositif d'analyse de gaz D2 permet de déterminer la composition du gaz circulant dans les lignes principale 10 et secondaire 20, en aval des capacités B, C, c'est-à-dire qu'il permet d'analyser en discontinu le gaz issu des capacités B et C.

Comme précédemment, ce deuxième dispositif d'analyse de gaz D2 coopère avec le dispositif de pilotage 4 qui lui-même commande les électrovannes V5, V6 via des liaisons électriques 37.

L'alimentation électrique de l'installation 100 est réalisée de manière classique par du courant du secteur, par exemple à une tension entre 1 et 600 V, typiquement 24 V, 230 V ou 400 V.

Si besoin est, des moyens de mesure (non montrés) peuvent être prévus pour déterminer la pression de l'air médical contenu dans les capacités A, B, C de stockage et d'homogénéisation du gaz et éventuellement rétroagir, via le dispositif de pilotage 4, sur l'unité de compression 31 et/ou l'unité de production 50 de sorte à réguler la production du gaz, tel l'air, en prenant en compte la (ou les) pression ainsi mesurées. Par exemple, les moyens de pilotage 4 peuvent être programmés pour commander l'arrêt de la source de débit 31 et/ou le déclenchement d'une alarme sonore et/ou visuelle, lorsqu'un capteur de pression, agencé au niveau de la capacité tampon A, détecte une pression ou une différence de pression supérieure ou, à l'inverse, inférieure à une valeur-seuil préfixée. Ce type de régulation en pression est parfaitement connu et ne sera pas détaillé ici.

En outre, afin d'assurer une purification encore plus efficace de l'air aspiré par le compresseur 31, on peut également prévoir un (ou plusieurs) dispositif de filtration mécanique (non détaillé) agencé en un ou plusieurs sites, entre la source d'air 31 et le réseau hospitalier 30. Par exemple, l'unité de compression peut comprendre un ou des filtres en entrée 32 et/ou en sortie 33 pour retenir les poussières contenues dans l'air ambiant et les condensats dues à la compression, par exemple des filtres ou séparateurs cycloniques, des filtres microniques ou analogues.

De façon générale, l'installation 100 de production sur site d'air médical, c'est-à-dire au sein d'un bâtiment hospitalier ou analogue, de l'invention met donc en oeuvre une électrovanne à trois voies apte à évacuer vers l'atmosphère, du gaz produit contaminé par des impuretés, par l'intermédiaire d'une ligne de purge reliée à l'une des voies de l'électrovanne VA en réponse à une détection de ladite contamination par un dispositif d'analyse D1 coopérant avec un dispositif de pilotage 4.

L'installation de production 100 d'air médical de l'invention est utilisable directement sur site d'utilisation du gaz, c'est-à-dire directement au sein d'un bâtiment hospitalier ou analogue. Elle peut donc être installée directement dans une pièce du bâtiment hospitalier ou alors à l'extérieur dudit bâtiment ou dans des conteneurs et reliée au réseau 30 de canalisations acheminant le gaz à l'intérieur de celui-ci.

Comme déjà mentionné et illustré sur la Figure 2, la commande de l'envoi du gaz stocké dans les capacités B et C vers le ou les sites utilisateurs 30 se fait via un procédé de pilotage mettant en oeuvre une fourniture alternée du gaz comprenant les étapes de :
- alimenter le site utilisateur 30 avec du gaz purifié, c'est-à-dire ici de l'air médical, ayant une composition souhaitée, c'est-à-dire conforme à la pharmacopée européenne, provenant de la troisième capacité C,
- alimenter simultanément ou quasi-simultanément la deuxième capacité B avec du gaz provenant de la première capacité A jusqu'à remplissage de la deuxième capacité B, puis stopper alors l'alimentation de la deuxième capacité B,
- analyser la composition du gaz stocké dans la deuxième capacité B, notamment au moyen du deuxième dispositif d'analyse de gaz D2, pour déterminer si la composition du gaz stocké dans la deuxième capacité B est conforme à la composition souhaitée, notamment si elle contient une proportion d'impuretés indésirables du type vapeur d'eau, vapeurs d'huile, SOx, COx et NOx, supérieure à une valeur maximale ou valeur-seuil donnée, par exemple les valeurs maximales tolérées fixées par la pharmacopée ou des valeurs inférieures,
- et en fonction du résultat de l'analyse, envoyer le gaz stocké dans la deuxième capacité B soit vers l'atmosphère lorsque la composition analysée n'est pas conforme à la composition souhaitée, soit vers le site utilisateur 30 lorsque la composition analysée est conforme à la composition souhaitée de manière à alimenter ledit site utilisateur 30 avec du gaz stocké dans la deuxième capacité B, après arrêt de l'alimentation dudit site utilisateur 30 avec du gaz issu de la troisième capacité C.

Lorsque la composition analysée n'est pas conforme à la composition souhaitée, on vide et on purge totalement ou quasi-totalement la deuxième capacité B et on répète ensuite la séquence d'étapes sus-décrite.

En d'autres termes, les deuxième B et troisième C capacités sont alternativement soumises aux étapes ci-avant, ce qui permet de contrôler précisément la qualité du gaz qu'elles contiennent et d'éviter ainsi que du gaz non conforme ne soit envoyé au site utilisateur 30, tel un réseau de canalisations au sein d'un bâtiment hospitalier ou similaire.

Afin de mettre en oeuvre les étapes ci-avant, le dispositif de pilotage 4 agit sur les vannes VA, V1-V8 qui sont agencées sur la ligne principale de gaz 10, la ligne secondaire 20, sur la première ligne de purge 13 et la deuxième ligne de purge 14, ainsi que sur la ligne de mesure 36, 36a, 36b.

Préférentiellement, le gaz produit est de l'air médical ou de l'oxygène médical, de préférence de l'air médical.

Ces étapes sont davantage détaillées sur la Figure 2 qui met en évidence le cycle de remplissage, analyse et libération de l'air produit, qui est opéré via les capacités B et C, les électrovannes V1 à V8 contrôlées par l'automate 4 et la baie d'analyse D2 qui elle-même coopère avec l'automate 4.

Plus précisément, sur la Figure 2, on comprend que :
- après démarrage 200 du cycle la situation de départ est la suivante : le contenu gazeux de la capacité C est analysé et libéré 201 pour approvisionner le réseau 30 de l'hôpital en gaz médical, tel de l'air. La vanne V4 est alors ouverte et la capacité B est vide. Les vannes V1 à V3 et V5 à V8 sont fermées.
- on procède alors à un remplissage de la capacité B par ouverture 202 de la vanne V1, avec de l'air purifié venant de la première capacité A (cf. Figure 1) jusqu'à remplir totalement la capacité B.
- lorsque la capacité B est pleine 203, c'est-à-dire après une durée de remplissage donnée, on ferme la vanne V1 et on ouvre la vanne V5 (en 204).
- on analyse ensuite (en 205) le contenu de la capacité B. A la fin de l'analyse, on opère une fermeture de la vanne V5 (en 206).
- si les résultats d'analyse (en 207) sont conformes (« oui ») aux spécifications souhaitées pour le gaz produit, le contenu de la capacité B est libéré mais reste en attente jusqu'à ce que la capacité C soit totalement vide (211).
- à l'inverse, si les résultats d'analyse ne sont pas conformes (« non ») aux spécifications, la vanne V7 est ouverte 208 et le contenu de la capacité B est évacué 209 vers l'atmosphère, via la ligne d'échappement 11 de la Figure 1 comme déjà expliqué. Lorsque la capacité B est vide, la vanne V7 est fermée 210 et le cycle de remplissage redémarre en 202 comme précédemment jusqu'à obtenir un résultat d'analyse (en 207) conforme.

En 211, lorsque la capacité C est vide, la vanne V4 est fermée et la vanne V2 est ouverte (en 212). La capacité B alimente 213 alors à son tour le réseau hôpital 30 en air médical.

En parallèle, il se produit (en 214) un remplissage de la capacité C avec du gaz médical par ouverture de la vanne V3 et ce, jusqu'à obtenir une capacité C pleine (en 215).

Alors, de manière similaire à ce qui a été opéré sur la capacité B, on opère une analyse 217 du contenu de la capacité C, et l'automate 4 commande une fermeture de la vanne V3 et une ouverture de la vanne V6 (en 216).

A la fin de l'analyse, l'automate 4 commande la fermeture 218 de la vanne V6.

A nouveau, si les résultats d'analyse sont conformes (« oui ») aux spécifications (en 219), le contenu de la capacité C est libéré et reste en attente jusqu'à ce que la capacité B soit vide (en 223).

Par contre, si les résultats d'analyse ne sont pas conformes (« non ») aux spécifications, la vanne V8 est ouverte 220 et le contenu de la capacité C est évacué vers l'extérieur, via l'échappement 12 et la ligne de purge 11 de la Figure 1. Lorsque la capacité C est vide 221, la vanne V8 est fermée 222 et le cycle de remplissage redémarre en 214

Lorsque la capacité B est vide, l'automate 4 commande une fermeture de la vanne V2 et une ouverture de la vanne V4. La capacité B alimente (en 224) alors à son tour le réseau hôpital 30 en air médical.

Enfin, le cycle redémarre au début 225.

Ce cycle est particulièrement efficace car il évite d'assécher le (ou les) site utilisateur 30, même lorsqu'une contamination du gaz est détectée, c'est-à-dire un taux d'impuretés supérieur aux spécifications désirées, grâce à une fourniture d'air ou de gaz médical audit site utilisateur 30 par alternance, c'est-à-dire en provenance soit de la capacité B, soit de la capacité C.

Ceci permet non seulement d'éviter de contaminer le site utilisateur 30 avec du gaz non conforme mais aussi de minimiser le recours à de l'air provenant d'une source de secours 3 (cf. Fig. 1).

## Revendications

1. Installation de production (100) sur site de gaz médical comprenant :
- une unité de purification de gaz (50) apte à produire un gaz purifié à partir d'un gaz d'alimentation,
- une ligne principale de gaz (10) alimentée en gaz par l'unité de purification de gaz (50) et comprenant, en série, une première capacité (A) de stockage de gaz et une deuxième capacité (B) de stockage de gaz purifié, et
- une ligne secondaire (20) connectée fluidiquement à la ligne principale (10), en aval de la première capacité (A) de stockage de gaz, et comprenant une troisième capacité (C) de stockage de gaz,
- au moins un dispositif d'analyse de gaz (D2) agencé de manière à déterminer la teneur du gaz en au moins une impureté donnée, en aval de la deuxième (B) et de la troisième (C) capacité,
- une première électrovanne (V1) agencée sur la ligne principale de gaz (10) entre la première capacité (A) de stockage de gaz et la deuxième capacité (B) de stockage de gaz,
- une deuxième électrovanne (V3) agencée sur la ligne secondaire de gaz (20) entre la première capacité (A) et la troisième capacité (C) de stockage de gaz,
- une troisième électrovanne (V2) agencée sur la ligne principale de gaz (10) en aval de la deuxième capacité (B) de stockage de gaz,
- une quatrième électrovanne (V4) agencée sur la ligne secondaire de gaz (20) en aval de la troisième capacité (C) de stockage de gaz, et
- un dispositif de pilotage (4) commandant l'ouverture ou la fermeture desdites électrovannes (V1 à V4) en réponse à au moins un signal de mesure de teneur en impureté reçu dudit au moins un dispositif d'analyse de gaz (D2).

2. Installation selon la revendication précédente, **caractérisée en ce qu'**une électrovanne à trois voies (VA) est agencée sur la ligne principale de gaz (10) entre l'unité de purification de gaz (50) et la première capacité (A) de stockage, de préférence l'électrovanne à trois voies (VA) est connectée à l'atmosphère (en 12) via une ligne d'échappement (11), et est commandée par le dispositif de pilotage (4).

3. Installation selon l'une des revendications précédentes, **caractérisée en ce qu'**elle comporte un premier dispositif d'analyse de gaz (D1) ayant une première ligne de mesure (29) raccordée fluidiquement (en 28) à la ligne principale (10), en amont de la première capacité de stockage (A).

4. Installation selon l'une des revendications précédentes, **caractérisée en ce qu'**elle comporte un deuxième dispositif d'analyse de gaz (D2) ayant au moins une deuxième ligne de mesure (36 ; 36a ; 36b) raccordée fluidiquement (en 34,35), directement ou indirectement, à la ligne principale (10) et à la ligne secondaire (20), en aval de la deuxième capacité de stockage (B) et de la troisième capacité de stockage (C), respectivement.

## Patentansprüche

1. Anlage zur Vor-Ort-Erzeugung (100) von medizinischem Gas, umfassend:
- eine Gasreinigungseinheit (50), die imstande ist, ein gereinigtes Gas ausgehend von einem Versorgungsgas zu erzeugen,
- eine Gashauptleitung (10), die von der Gasreinigungseinheit (50) mit Gas versorgt wird und, in Reihe, eine erste Speicherkapazität (A) für Gas und eine zweite Speicherkapazität (B) für gereinigtes Gas umfasst, und
- eine Nebenleitung (20), die fluidisch mit der Hauptleitung (10) verbunden ist, stromabwärts von der ersten Speicherkapazität (A) für Gas, und eine dritte Speicherkapazität (C) für Gas umfasst,
- mindestens eine Gasanalysevorrichtung (D2), die angeordnet ist, um den Gehalt des Gases an mindestens einer gegebenen Verunreinigung zu bestimmen, stromabwärts von der zweiten (B) und der dritten (C) Kapazität,
- ein erstes Magnetventil (V1), das auf der Gashauptleitung (10) zwischen der ersten Speicherkapazität (A) für Gas und der zweiten Speicherkapazität (B) für Gas angeordnet ist,
- ein zweites Magnetventil (V3), das auf der Gasnebenleitung (20) zwischen der ersten Kapazität (A) und der dritten Speicherkapazität (C) für Gas angeordnet ist,
- ein drittes Magnetventil (V2), das auf der Gashauptleitung (10) stromabwärts von der zweiten Speicherkapazität (B) für Gas angeordnet ist,
- ein viertes Magnetventil (V4), das auf der Gasnebenleitung (20) stromabwärts von der dritten Speicherkapazität (C) für Gas angeordnet ist, und
- eine Steuervorrichtung (4), die die Öffnung oder die Schließung der Magnetventile (V1 bis V4) als Reaktion auf mindestens ein Signal zur Messung eines Verunreinigungsgehalts regelt, das von der mindestens einen Gasanalysevorrichtung (D2) empfangen wird.

2. Anlage nach dem vorstehenden Anspruch, **dadurch gekennzeichnet, dass** ein Drei-Wege-Magnetventil (VA) auf der Gashauptleitung (10) zwischen der Gasreinigungseinheit (50) und der ersten Speicherkapazität (A) angeordnet ist, vorzugsweise ist das Drei-Wege-Magnetventil (VA) mit der Atmosphäre (in 12) über eine Abgasleitung (11) verbunden und durch die Steuervorrichtung (4) geregelt.

3. Anlage nach einem der vorstehenden Ansprüche, **dadurch gekennzeichnet, dass** sie eine erste Gasanalysevorrichtung (D1) umfasst, die eine erste Messleitung (29) aufweist, die fluidisch (in 28) an die Hauptleitung (10) angeschlossen ist, stromaufwärts von der ersten Speicherkapazität (A).

4. Anlage nach einem der vorstehenden Ansprüche, **dadurch gekennzeichnet, dass** sie eine zweite Gasanalysevorrichtung (D2) umfasst, die mindestens eine zweite Messleitung (36; 36a; 36b) aufweist, die fluidisch (in 34,35), direkt oder indirekt, an die Hauptleitung (10) und an die Nebenleitung (20) angeschlossen ist, jeweils stromabwärts von der zweiten Speicherkapazität (B) und der dritten Speicherkapazität (C).

## Claims

1. Installation for the production (100) on site of medical gas comprising:
- a gas purification unit (50) capable of producing a purified gas from a feed gas,
- a main gas line (10) supplied with gas by the gas purification unit (50) and comprising, in series, a first storage capacity (A) for gas and a second storage capacity (B) for purified gas, and
- a secondary line (20) fluidically connected to the main line (10), downstream of the first storage capacity (A) for gas, and comprising a third storage capacity (C) for gas,
- at least one gas analysis device (D2) arranged so as to determine the content of the gas in at least one given impurity, downstream of the second (B) and of the third (C) capacity,
- a first solenoid valve (V1) arranged on the main gas line (10) between the first storage capacity (A) for gas and the second storage capacity (B) for gas,
- a second solenoid valve (V3) arranged on the secondary gas line (20) between the first capacity (A) and the third storage capacity (C) for gas,
- a third solenoid valve (V2) arranged on the main gas line (10) downstream of the second storage capacity (B) for gas,
- a fourth solenoid valve (V4) arranged on the secondary gas line (20) downstream of the third storage capacity (C) for gas, and
- a control device (4) controlling the opening or the closing of said solenoid valves (V1 to V4) in response to at least one signal for measuring the content in impurity received from said at least one gas analysis device (D2).

2. Installation according to the preceding claim, **characterised in that** a three-way solenoid valve (VA) is arranged on the main gas line (10) between the gas purification unit (50) and the first storage capacity (A), preferably the three-way solenoid valve (VA) is connected to the atmosphere (in 12) via an exhaust line (11), and is controlled by the control device (4).

3. Installation according to one of the preceding claims, **characterised in that** it comprises a first gas analysis device (D1) having a first measurement line (29) fluidically connected (in 28) to the main line (10), upstream of the first storage capacity (A).

4. Installation according to one of the preceding claims, **characterised in that** it comprises a second gas analysis device (D2) having at least one second measurement line (36; 36a; 36b) fluidically connected (in 34, 35), directly or indirectly, to the main line (10) and to the secondary line (20), downstream of the second storage capacity (B) and of the third storage capacity (C), respectively.
